# EUROPEAN PATENT APPLICATION

(11) **EP 1 882 941 A2**
(43) Date of publication of application: **30.01.2008**
(21) Application number: 07108655.7
(22) Date of filing: 22.05.2007
(51) Int. Cl.: G01N 33/543, G01N 35/00, C12N 15/10

(54) **Magnetic bead extraction device for separating and purifying target biomolecules and microfluidic system including the same**

(30) Priority: 25.07.2006 KR 20060069496
(71) Applicant: Samsung Electronics Co., Ltd., Suwon-si, Gyeonggi-Do (KR)
(72) Inventor: Cho, Yoon-kyoung c/o Samsung Advanced Institute of Techn., Gyeonggi-do (KR); Lee, Jeong-gun c/o Samsung Advanced Inst. of Techn., Gyeonggi-do (KR); Lee, Beom-seok c/o Samsung Advanced Inst. of Techn., Gyeonggi-do (KR); Park, Jong-myeon c/o Samsung Advanced Inst. of Techn., Gyeonggi-do (KR); Hong, Sung-woo c/o Samsung Advanced Inst. of Techn., Gyeonggi-do (KR)
(74) Representative: Grünecker, Kinkeldey, Stockmair & Schwanhäusser Anwaltssozietät

(57) **Abstract**

Provided are a magnetic bead extraction device for separating and purifying biomolecules and a microfluidic system using the magnetic bead extraction device. The magnetic bead extraction device includes a buffer solution chamber, a sample solution channel including an inlet, an outlet, a flow channel formed between the inlet and the outlet for allowing a sample solution to flow therethrough, and a connecting portion formed in a portion of the flow channel and connected to the buffer solution chamber, and a magnetic body disposed close to the buffer solution chamber to apply a magnetic force to magnetic beads contained in the sample solution so as to attract the magnetic beads into the buffer solution chamber through the connecting portion. Therefore, in the magnetic bead extraction device, only the magnetic beads can be collected into the buffer solution chamber from the sample solution flowing through the sample solution channel, and the remaining sample solution can be discharged through the outlet of the sample solution channel, thereby purifying the magnetic beads without an additional purification process.

## Description

### BACKGROUND OF THE INVENTION

### 1. Field of the Invention

The present invention relates to a device for extracting magnetic beads coupled with target biomolecules from a sample solution so as to separate and purify the target biomolecules, and a microfluidic system including the device.

### 2. Description of the Related Art

Target biomolecules can be separated from biological samples such as blood plasma using silica, glass fibers, anion exchange resins, or magnetic beads. In a biomolecule separating method using magnetic beads, magnetic beads having probes on their surfaces are mixed with a sample solution to capture target biomolecules using the probes formed on the magnetic beads, and then the magnetic beads coupled with target biomolecules are separated from the sample solution, thereby extracting the target biomolecules. This biomolecule separating method (known as bead based separation) is already commercially used for separating biomolecules such as cells, proteins, and nucleic acids. For example, U.S. Patent No. 6,893,881 discloses a method of separating desired target cells using antibody-coated paramagnetic beads.

When target biomolecules are separated using magnetic beads, bead washing is necessary. For example, when antibodies are coupled to surfaces of magnetic beads for capturing antigens using the antibodies coupled to the magnetic beads, remaining antibodies not coupled to the magnetic beads and other unnecessary substances should be removed by washing the magnetic beads. Further, after a sample solution such as blood and serum is mixed with magnetic beads and is processed (e.g., incubated), substances such as remaining blood plasma and serum should be removed so as to prevent undesired results in the following operations. That is, washing is necessary in this case.

In a conventional washing process, a vessel containing a mixture of a sample solution and magnetic beads is placed close to a magnet so as to attract the magnetic beads towards a wall of the vessel located adjacent to the magnet, and then the remaining sample solution is removed using a pipette. These procedures are repeated while adding a washing buffer solution until the mixture is washed to a desired level. That is, the vessel is taken away from the magnet, and a washing buffer solution is added into the vessel. Then, the vessel is placed close to the magnet again, and the remaining solution is removed.

In this case, since it is difficult to remove a desired amount of solution at a time, the washing procedures should be repeated two or more times, requiring a lot of time and effort. Furthermore, since a large amount of buffer solution is required, it is practically impossible to perform such a washing process for a microchip. Moreover, when the amount of sample solution is several micro liters, it is difficult to remove the sample solution using a pipette, making it difficult to precisely control the quantity of the sample solution or the buffer solution.

### SUMMARY OF THE INVENTION

It is an object of the present invention to provide a magnetic bead extraction device that can effectively and rapidly separate and purify magnetic beads coupled with target biomolecules from a sample solution using a small amount of buffer solution, and a microfluidic system including the same.

This object is solved by the features of claim 1.

The flow channel of the sample solution channel may be V-shaped, and the connecting portion of the sample solution channel may be located at a tip of the V-shaped flow channel. While flowing through the connecting portion of the sample solution channel, the sample solution may approach a buffer solution contained in the buffer solution chamber at an angle greater than 0° but less than 90° and may depart from the buffer solution at an angle greater than 0° but less than 90°. The magnetic body may be disposed at a lower or upper outside portion of the buffer solution chamber.

The buffer solution chamber may contain a buffer solution, and the buffer solution may be stationary in the buffer solution chamber when the magnetic bead extraction device operates.

The sample solution channel may have a width and height and may be bent at an angle such that the sample solution flows through the sample solution channel and passes through the connecting portion in a laminar state when the magnetic bead extraction device operates. The reason for this is to prevent substances other than the magnetic beads from mixing with the buffer solution. The substances may be discharged through the outlet of the sample solution channel together with the laminar flow of the sample solution.

It is possible to use a buffer solution chamber including an inlet, an outlet, and a flow channel formed between the inlet and the outlet for allowing a buffer solution to flow therethrough.

The width and height of the buffer solution chamber may be such that the buffer solution flows through the flow channel of the buffer solution chamber in a laminar state when the magnetic bead extraction device operates. The sample solution and the buffer solution may flow in the same direction at the connecting portion.

According to a further another aspect of the present invention, there is provided a microfluidic system for separating target biomolecules from a sample solution using magnetic beads, the microfluidic system including at least one magnetic bead extraction unit as disclosed above separating and purifying magnetic beads from a sample solution.

The magnetic beads may have a diameter in the range of 0.001 µm to 200 µm. The magnetic beads may have a surface layer on which an unspecific biomolecule can be attached. For this, the surface layer of the magnetic bead may be formed of a metal oxide, styrene, agarose, or silica. Alternatively, the surface layer of the magnetic beads may be formed with a probe capable of coupling with a particular target molecule. In this case, the probe may be one selected from the group consisting of an antibody, an antigen, a DNA, biotin, a protein, and streptavidin, or the probe may include an amino radical (NH₂-) or a carboxyl radical (COOH-).

Herein, the term biomolecule is used to denote a biosynthetic molecule such as an amino acid, a protein, sugar, lipid, and a nucleic acid, plus an animal cell, a virus, and bacteria.

### BRIEF DESCRIPTION OF THE DRAWINGS

The above and other features and advantages of the present invention will become more apparent by describing in detail exemplary embodiments thereof with reference to the attached drawings in which:
FIG. 1 is a plan view illustrating a magnetic bead extraction device according to an embodiment of the present invention;
FIG. 2 is a sectional view taken along a line II-II of FIG. 1, according to an embodiment of the present invention;
FIG. 3A is a photographic image illustrating an initial stage of a process of extracting magnetic beads using the magnetic bead extraction device of FIG. 1, according to an embodiment of the present invention;
FIG. 3B is a photographic image illustrating magnetic beads extracted using the magnetic bead extraction device of FIG. 1, according to an embodiment of the present invention;
FIG. 4 is a graph illustrating results of real-time polymerase chain reaction (PCR) for a comparison example;
FIG. 5 is a graph illustrating results of real-time PCR for a sample separated and purified using the magnetic bead extraction device of FIG. 1, according to an embodiment of the present invention; and
FIG. 6 is a plan view illustrating a microfluidic system according to an embodiment of the present invention.

### DETAILED DESCRIPTION OF THE INVENTION

The present invention will now be described more fully with reference to the accompanying drawings, in which exemplary embodiments of the invention are shown.

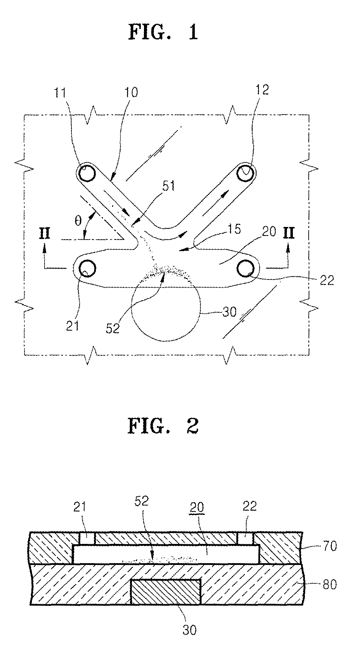

FIG. 1 is a plan view illustrating a magnetic bead extraction device according to an embodiment of the present invention. Referring to FIG. 1, the magnetic bead extraction device according to the current embodiment of the present invention includes a sample solution channel 10 and a buffer solution chamber 20. The sample solution channel 10 provides a passage for a sample solution containing magnetic beads 51, and the buffer solution chamber 20 temporarily stores a buffer solution and allows the buffer solution to flow therethrough. The sample solution channel 10 includes an inlet 11 and an outlet 12 to receive and discharge a sample solution, a flow channel formed between the inlet 11 and the outlet 12 to allow the sample solution to flow from the inlet 11 to the outlet 12, and a connecting portion 15 formed in the middle of the flow channel and connected to the buffer solution chamber 20. The sample solution channel 10 can be bent at the connecting portion 15. For example, the sample solution channel 10 can be bent into a V shape. The buffer solution chamber 20 includes an inlet 21 and an outlet 22 to receive and discharge a buffer solution. A magnetic body 30 is disposed close to a top or bottom portion of the buffer solution chamber 20. The magnetic body 30 applies a magnetic force to a sample solution passing through the connecting portion 15 such that the magnetic beads 51 contained in the sample solution can be attracted to the buffer solution chamber 20.

The sample solution channel 10 and the buffer solution chamber 20 make an angle θ. The angle θ may be determined within a range greater than 0° but less than 90°. When the angle θ is excessively small, the magnetic beads 51 may flow together with the sample solution toward the outlet 12 past the connecting portion 15 instead of being attracted toward the magnetic body 30. On the other hand, when the angle θ is excessively large, the sample solution as well as the magnetic beads 51 may flow into the buffer solution chamber 20 through the connecting portion 15. In the current embodiment, the sample solution channel 10 is connected to the buffer solution chamber 20 at the connecting portion 15 in such a manner that the flow channel formed in the sample solution channel 10 approaches and departs from the buffer solution filled in the buffer solution chamber 20 at an angle of 45°. The width of the connecting portion 15 may be determined such that the magnetic beads 51 can flow into the buffer solution chamber 20 through the connecting portion 15. For this, the width of the connecting portion 15 may be determined in consideration of the flow rate of the sample solution in the sample solution channel 10, the magnetic force of the magnetic body 30, the size of the magnetic beads 51, etc. Further, the connecting portion 15 is properly designed such that material diffusion can be reduced between the sample solution and the buffer solution.

The buffer solution chamber 20 may temporarily store buffer solution when the magnetic bead extraction device operates. Alternatively, like the sample solution channel 10, the buffer solution chamber 20 may include a flow channel formed between the inlet 21 and the outlet 22 to allow buffer solution to flow therethrough.

The magnetic bead extraction device can be formed in a chip as a part of a microfluidic system. Specifically, the magnetic bead extraction device can be formed by engraving (patterning) a sample solution channel 10 and a buffer solution chamber 20 (referring to FIG. 1) in an inner surface of one of stacked two plates, and by forming holes in the plate as inlets 11 and 21 and outlets 12 and 22.

FIG. 2 is a sectional view of the magnetic bead extraction device taken along a line II-II of FIG. 1, according to an embodiment of the present invention. In the current embodiment of the present invention, the magnetic bead extraction device is formed in a chip. In detail, an engraved pattern is formed in a bottom surface of an upper plate 70 to a predetermined depth so as to form the buffer solution chamber 20, and the inlet 21 and outlet 22 are formed at both sides of the buffer solution chamber 20. The magnetic body 30 is formed in a lower plate 80 close to a bottom portion of the buffer solution chamber 20.

The magnetic bead extraction device according to the current embodiment of the present invention and a microfluidic system using the magnetic bead extraction device are advantageous, for example, in realizing integrated lab-on-a-chips that use magnetic beads for separating target cells or viruses from liquid having a complicated composition such as whole blood, saliva, and urine, purifying the separated target cells or viruses, and rapidly separating nucleic acid from the purified target cells or viruses.

For instance, when biotin-coupled cells or virus-specific antibodies react with streptavidin-conjugated magnetic beads, the antibodies are coupled to the magnetic beads owing to the affinity between the streptavidin and the biotin. When the antibody-coupled magnetic beads are mixed with a sample solution containing cells or viruses, antibodies coupled to the magnetic beads combine with specific cells or viruses. In this way, specific cells or viruses can be concentrated. Therefore, desired cells or viruses can be concentrated using antibodies capable of coupling with the desired cells or viruses. After that, desired DNAs can be obtained by breaking the concentrated cells or viruses using various cell-breaking methods.

In the microfluidic system according to an embodiment of the present invention, the surfaces of the magnetic beads can be formed of a material selected from the group consisting of metal oxide, styrene, agarose, and silica so as to combine with unspecific biomolecules. Alternatively, probes capable of combining with specific target biomolecules can be formed on the surfaces of the magnetic beads. Such a probe causing the magnetic beads to have an affinity for specific target molecules may be one of an antibody, an antigen, a DNA, biotin, and streptavidin or may be formed of a material having an amino radical (NH₂-) or a carboxyl radical (COOH-). For example, when the magnetic beads are surface treated with antibodies, very low-density cells or viruses can be easily detected since the antibodies couple with a specific kind of cell or virus but do not couple with others. Magnetic beads treated with antibodies capable of coupling with specific cells or viruses are commercially sold by companies such as Invitrogen and Qiagen. Examples of such commercially available magnetic beads include Dynabeads® Genomic DNA Blood (Invitrogen), Dynabeads® anti-E.coli 0157(Invitrogen), CELLection™ Biotin Binder Kit (Invitrogen), and MagAttract Virus Min M48 Kit(Qiagen). For example, the magnetic beads treated with antibodies can be used to separate the following: Diphtheria toxin, Enterococcus faecium, Helicobacter pylori, HBV, HCV, HIV, Influenza A, Influenza B, Listeria, Mycoplasma pneumoniae, Pseudomonas sp., Rubella virus, and Rotavirus.

In the microfluidic system according to an embodiment of the present invention, the size (diameter) of the magnetic beads may be in the range of 0.001 µm to 200 µm. More specifically, the size of the magnetic beads may be in the range of 0.1 µm to 100 µm. The size of the magnetic beads may be properly selected according to the size of target biomolecules to be separated and purified using the microfluidic system. Further, the magnetic beads can be formed of any magnetic material. In particular, the magnetic beads can be formed of at least one material selected from the group consisting of ferromagnetic Fe, Ni, Cr, and oxides thereof.

An operation of the magnetic bead extraction device of FIGS. 1 and 2 will now be described to provide clearer understanding of the characteristic features of the present invention.

When a sample solution containing magnetic beads 51 is filled into the sample solution channel 10 through the inlet 11 in a state where a buffer solution filled in the buffer solution chamber 20 is stationary, the sample solution passes through the connecting portion 15 as it flows through the flow channel of the sample solution channel 10. When the sample solution passes through the connecting portion 15, the magnetic beads 51 contained in the sample solution are attracted toward the magnetic body 30 by a magnetic force of the magnetic body 30, and thus the magnetic beads 51 are separated from the sample solution and are mixed with the buffer solution in the buffer solution chamber 20. Therefore, the magnetic beads 51 can be collected in the buffer solution chamber 20. Meanwhile, the sample solution separated from the magnetic beads 51 flows from the connecting portion 15 to the outlet 12. In this way, the magnetic beads 51 can be nearly completely separated from the sample solution at one time. That is, the magnetic beads 51 can be purified using a very small amount of buffer solution such that an additional purification process is not required for the magnetic beads 51 (hereinafter, the purified magnetic beads will be denoted using reference numeral 52). Here, the flow of the sample solution in the sample solution channel 10 may remain laminar. In this case, material diffusion from the laminar flow of the sample solution to the stationary buffer solution may be negligibly small.

When the buffer solution chamber 20 includes a flow channel formed from the inlet 21 to the outlet 22 (i.e., when a buffer solution flows through a center region of the connecting portion 15), the magnetic beads 51 can be separated from the sample solution flowing along the sample solution channel 10 in the same way as described above. In this case, some of the sample solution can flow into the buffer solution chamber 20 together with the magnetic beads 51. However, the sample solution can be discharged through the outlet 22 of the buffer solution chamber 20, so that only the purified magnetic beads 52 can remain in the buffer solution chamber 20. In fact, the amount of sample solution introduced into the buffer solution chamber 20 through the connecting portion 15 may be very small. Therefore, like in the case where the buffer solution is stationary in the buffer solution chamber 20, purified magnetic beads 52 can be obtained using a very small amount of buffer solution as compared with a conventional method.

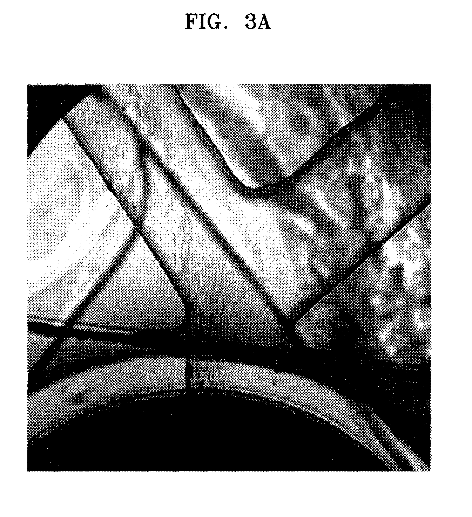

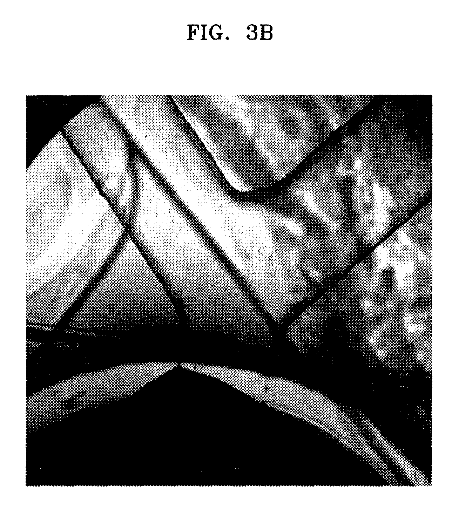

FIG. 3A is a photographic image illustrating an initial stage of a process of extracting magnetic beads using the magnetic bead extraction device of FIG. 1, according to an embodiment of the present invention, and FIG. 3B is a photographic image illustrating magnetic beads extracted using the magnetic bead extraction device of FIG. 1, according to an embodiment of the present invention. Referring to FIGS. 3A and 3B, as described above, magnetic beads are attracted toward a magnetic body from a flow of sample solution. FIG. 3B shows magnetic beads collected in a buffer solution chamber after two minutes of sample solution flow.

The following examples are for illustrative purposes only and are not intended to limit the scope of the present invention.

### <EXPERIMENTAL EXAMPLE: Separation and Purification of HBV using Magnetic Bead Extraction Device of the present invention>

### 1) Preparation of Hepatitis B virus (HBV), secondary antibody, and magnetic bead

100 µl of whole blood containing 10³ to 10⁶ HBV infected cells was prepared. A 10 µl solution of biotin-coupled secondary antibody (Virostat, 1817, host animal: rabbit) was prepared. 20 µl of Dynabeads®M-280 Streptavidin (streptavidin-labeled, 2.8-µm-diameter, magnetic beads) was prepared.

### 2) Washing of beads

A homogeneous bead solution was prepared by mixing beads with a predetermined solution. 100 µl of the homogeneous bead solution was filled into a tube, and the tube was placed on a magnet for two minutes. Then, an upper layer of the solution was removed using a pipette, leaving the beads attracted by the magnet. After that, the tube was taken away from the magnet, and 100 µl of buffer solution (PBS containing 0.1 % BSA, pH 7.4) was added into the tube where the settled beads remained. Then the tube was placed on the magnet again for two minutes. An upper layer of the buffer solution was removed using a pipette. In the same way, the tube was taken away from the magnet, and 100 µl of buffer solution (PBS containing 0.1 % BSA, pH 7.4) was added into the tube where the settled beads remained so as to obtain a bead solution containing washed beads.

### 3) Preliminary coating of beads with antibody

8 µg of biotin-coupled, anti-HBV, secondary antibody was mixed with a 100 µl bead solution prepared in operation 2. Next, the solution was incubated at room temperature for thirty minutes while rotating a vessel containing the solution several times. After that, beads contained in the solution were attracted down using a magnet, and an upper layer of the solution was removed. Next, 2 ml of washing buffer solution (PBS containing 1% BSA, pH 7.4) was added into the vessel where settled beads remained, and the vessel was rotated several times so as to mix the beads with the washing buffer solution. Next, the beads contained in the solution were attracted down using a magnet, and an upper layer of the solution was removed. In this way the beads were preliminarily coated with the HBV antibodies. Then, 100 µl of buffer solution (PBS containing 0.1 % BSA, pH 7.4) was added into the vessel where the preliminarily coated beads remained so as to obtain a suspension containing magnetic beads preliminarily coated with HBV antibodies.

### 4) Separation and purification of magnetic beads that have captured HBV

The HBV-antibody coated magnetic bead solution (suspension) obtained in operation 3 was mixed with the 100 µl HBV-infected whole blood prepared in operation 1. The mixture solution was incubated at a temperature of 2°C to 8°C for twenty minutes so as to allow the magnetic beads contained in the magnetic bead solution to capture the HBV contained in the whole blood. Next, the mixture solution was centrifuged, and an upper layer of the centrifuged mixture solution was removed until 160 µl of the centrifuged mixture was discarded. Then, the remaining mixture solution was passed through a magnetic bead extraction device similar to that of FIG. 1 at a flow rate of 10 µl/min for two minutes so as to separate and purify magnetic beads that captured the HBV.

### 5) Cell lysis

A 4 µl solution of HBV obtained from the HBV-coupled magnetic beads prepared in operation 4 was destructed by a cell destructing device using laser ablation (J.-G. Lee, K. H. Cheong, N. Huh, S. Kim, J.-W. Choi, C. Ko, Lab Chip 6, 886 (2006)). A real-time PCR process was performed on the destructed HBV. The real-time PCR process was performed using a GeneSpector® Micro PCR TMC-1000 (SAlT, Korea) (Y.-K. Cho, J. Kim, Y. Lee, Y.-A. Kim, K. Namkoong, H. Lim, K. W. Oh, S. Kim, J. Han, J. Park, Y. E. Pak, C.-S. Ki, J. R. Choi, H.-K. Myeong, C. Ko, Biosensors and Bioelectronics 21 (2006), 2161~2169).

### <COMPARISON EXAMPLES>

### Comparison Example A. Purified HBV solution sample

Purified HBV DNA was diluted with a PBS buffer solution to obtain an HBV solution having a concentration of 1×10³ cells/µl, and a real-time PCR polymerase chain reaction was directly performed using the HBV solution without a washing process.

### Comparison Example B. HBV solution sample washed by a conventional method

A HBV solution having a concentration of 1×10³ cells/µl was mixed with serum in the ratio of 1:3 by volume. A bead solution containing washed beads was obtained in a manner similar to operation "2)", and the bead solution was processed in a manner similar to operation "3)" to obtain an HBV-antibody coated magnetic bead solution (suspension). Then the HBV-antibody coated magnetic bead solution and the HBV-serum solution were mixed with each other and were incubated in a manner similar to operation "4)". After the incubation, instead of exacting beads from the incubated mixture solution using the magnetic bead extraction device of FIG. 1 as described in operation "4)", a conventional washing operation was performed. That is, beads were collected from the incubated mixture solution using a magnet for two minutes, and a supernatant of the solution was removed. Then, 100 µl of washing buffer solution was added to the collected beads and was mixed by rotating the mixture of the buffer solution and the beads several times. The beads were collected from the mixture solution using a magnet, and a supernatant of the mixture was removed. This operation was repeated three times to obtain washed magnetic beads that captured thereon. Then a cell-lysis process and a real-time PCR process were performed on the magnetic bead mixed solution sample.

### Comparison Example C. Unwashed HBV solution sample after mixed with serum

A HBV solution having a concentration of 1×10³ cells/µl was mixed with serum in the ratio of 1:3 by volume. A magnetic bead mixed solution sample was obtained through process B using this HBV-serum solution without a conventional washing operation, and a cell-lysis process and a real-time PCR process were performed on the unwashed magnetic bead mixed solution sample.

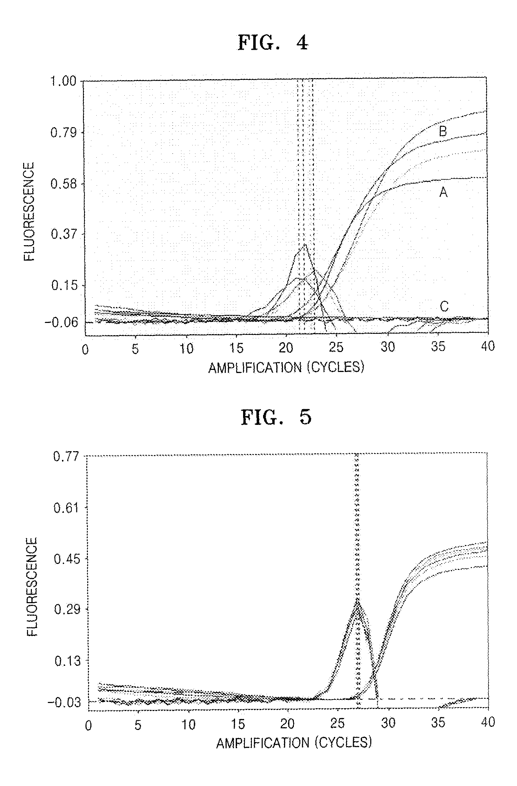

FIG. 4 is a graph illustrating real-time PCR results for the comparison examples. Table 1 below shows a summary of the real-time PCR result graph illustrated in FIG. 4.

**[Table 1]**

| SAMPLE TYPE | TARGET SEPARATION & WASHING STEP | Ct |
|---|---|---|
| PURIFIED HBV DNA (10³cells/µℓ) | X(A) | 21.79 |
| HBV(10³cells/µℓ):SE RUM MIXTURE (VOLUME RATIO 1:3) 100µℓ | O(B) | 22.22±0.77 |
| | X(C) | X |

Comparison Example A had a threshold cycle (Ct) of 21.79 and is used as a reference for the other examples. The threshold cycle (Ct) is the cycle at which a fluorescence signal is first detectable in a real time PCR (in other words, the threshold cycle (Ct) is equal to the number of cycles performed when the fluorescence signal is first detected in a real time PCR). That is, when the initial concentration of DNA is high, a fluorescence signal is first detected at a low threshold cycle (Ct). On the other hand, when the initial concentration of DNA is low, the fluorescence signal is first detected at a high threshold cycle (Ct). Further, the threshold cycle (Ct) relates to DNA purification. When the purification level of DNA is high, a fluorescence signal is first detected at a low threshold cycle (Ct), and when the purification level of DNA is low, the fluorescence signal is first detected at a high cycle. Therefore, it can be assumed that the purification level of DNA contained in a solution is high when the threshold cycle (Ct) has a low value.

The threshold cycle (Ct) of Comparison Example B was 22.22 (error range ±0.77), similar to that of Comparison Example A. However, PCR did not occur in the case of Comparison Example C where a washing process was not performed.

FIG. 5 is a graph illustrating results of real-time PCR for a sample separated and purified using the magnetic bead extraction device of FIG. 1, according to an embodiment of the present invention. The real-time PCR results shown in FIG. 5 were obtained in the same way as in the above-described experimental example. The same experiment was repeated six times to obtain reliable results. The measured threshold cycle (Ct) of the sample was 26.95 ±0.12. However, since the initial concentration of HBV in the sample was 1×10² cells/µl, the threshold cycle (Ct) of the sample cannot be directly compared with the threshold cycles (Ct) of Comparison Examples A and B in which the initial concentration of DNA was 1×10³ cells/µl. Therefore, it can be estimated that that the threshold cycle (Ct) of the sample may be about 23.65 (smaller than the measured threshold cycle (Ct) by 3.3) when the initial concentration of HBV in the sample is 1×10³ cells/µl. This estimation is based on the fact that the threshold cycle (Ct) relates to the initial concentration value of objects to be amplified by PCR (i.e., the higher the initial concentration value the lower threshold cycle (Ct) is). When the efficiency of PCR is 100%, a ten-times higher initial concentration value results in a reduction of the threshold cycle by 3.3 (ΔCt = 3.3).

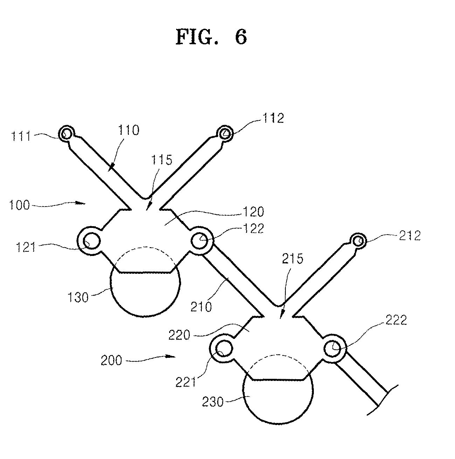

FIG. 6 is a plan view illustrating a microfluidic system according to an embodiment of the present invention. Referring to FIG. 6, the microfluidic system according to the current embodiment of the present invention can include at least one magnetic bead extraction unit corresponding to the magnetic bead extraction device illustrated in FIG. 1. When the microfluidic system includes a plurality of magnetic bead extraction units, the magnetic bead extraction units may be sequentially arranged so as to easily repeat separation and purification of magnetic beads. In FIG. 6, first and second magnetic bead extraction units 100 and 200 are exemplary illustrated.

The first and second magnetic bead extraction units 100 and 200 are sequentially disposed. That is, a buffer solution chamber outlet 122 of the first magnetic bead extraction unit 100 is connected to an inlet of a sample solution channel 210 of the second magnetic bead extraction unit 200. A valve (not shown) can be disposed between the buffer solution chamber outlet 122 of the first magnetic bead extraction unit 100 and the inlet of the sample solution channel 210 of the second magnetic bead extraction unit 200.

Hereinafter, an operation of the microfluidic system of FIG. 6 will be described to provide clearer understanding of the characteristic features of the present invention.

When a sample solution containing magnetic beads is filled into a sample solution channel 110 through an inlet 111 in a state where a buffer solution filled in a buffer solution chamber 120 of the first magnetic bead extraction unit 100 is stationary, the sample solution passes through a connecting portion 115 as it flows through the sample solution channel 110. When the sample solution passes through the connecting portion 115, the magnetic beads contained in the sample solution are attracted toward a magnetic body 130 by a magnetic force of the magnetic body 130, and thus the magnetic beads are separated from the sample solution and are mixed with the buffer solution in the buffer solution chamber 120. Therefore, the magnetic beads 51 can be collected in the buffer solution chamber 20. Meanwhile, the sample solution separated from the magnetic beads flows from the connecting portion 115 to an outlet 112 of the sample solution channel 110. In this way, the magnetic beads can be first purified. The magnetic body 130 may be detachably installed in the first magnetic bead extraction unit 100. The buffer solution containing the purified magnetic beads can be discharged from the buffer solution chamber 120 of the first magnetic bead extraction unit 100 after the magnetic body 130 is detached.

The buffer solution containing the purified magnetic beads discharged from the first magnetic bead extraction unit 100 is introduced into the sample solution channel 210 of the second magnetic bead extraction unit 200. A buffer solution chamber 220 of the second magnetic bead extraction unit 200 is also filled with a buffer solution, and the magnetic beads in the buffer solution introduced from the first magnetic bead extraction unit 100 can be purified in the same way as in the first magnetic bead extraction unit 100. Therefore, even when foreign substances diffuse into the buffer solution in the first magnetic bead extraction unit 100, the foreign substances can be discharged through an outlet 212 of the sample solution channel 210 of the second magnetic bead extraction unit 200. Therefore, the magnetic beads, which are collected into the buffer solution chamber 220 through a connecting portion 215 by a magnetic force of a magnetic body 230, can have a higher purification level.

Here, the flow of the sample solution may be laminar both in the sample solution channels 110 and 210 of the first and second magnetic bead extraction units 100 and 200. In this case, material diffusion from the flow of the sample solution into the stationary buffer solution can be minimized.

As described above, magnetic beads can be effectively separated from a sample solution using the magnetic bead extraction device in the microfluidic system according to the present invention. That is, magnetic beads on which target biomolecules are captured can be rapidly separated and purified using a small amount of buffer solution.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A magnetic bead extraction device (100, 200) comprising:
a buffer solution chamber (20, 120, 220);
a sample solution channel (10, 110, 210) comprising an inlet (11, 111), an outlet (12, 112, 212), a flow channel formed between the inlet and the outlet for allowing a sample solution to flow therethrough, and a connecting portion (15, 115, 215) formed in a portion of the flow channel and connected to the buffer solution chamber, and
a magnetic body (30, 130, 230) disposed close to the buffer solution chamber to apply a magnetic force to magnetic beads (51, 52) contained in the sample solution so as to attract the magnetic beads into the buffer solution chamber through the connection portion.

2. The magnetic bead extracting device according to claim 1, wherein said buffer solution chamber (20, 120, 220) comprises an inlet (21, 121, 221), an outlet (22, 122, 222), and a flow channel formed between the inlet and the outlet for allowing the buffer solution to flow therethrough.

3. The magnetic bead extraction device of claim 1 or 2, wherein the flow channel of the sample solution channel is V-shaped, and the connecting portion (15, 115, 215) of the sample solution channel is located at a tip of the V-shaped flow channel.

4. The magnetic bead extraction device of one of the previous claims, wherein, while flowing through the connecting portion of the sample solution channel, the sample solution approaches a buffer solution contained in the buffer solution chamber at an angle greater than 0° but less than 90° and departs from the buffer solution at an angle greater than 0° but less than 90°.

5. The magnetic bead extraction device of one of the previous claims, wherein the magnetic body (30, 130, 230) is disposed at a lower or upper outside portion of the buffer solution chamber (20, 120, 220).

6. The magnetic bead extraction device of one of the previous claims, wherein the buffer solution chamber (20, 120, 220) contains a buffer solution, and the buffer solution is stationary in the buffer solution chamber when the magnetic bead extraction device operates.

7. The magnetic bead extraction device of claim 6, wherein the sample solution channel (10, 110, 210) has a width and height and is bent at an angle such that the sample solution flows through the sample solution channel and passes through the connecting portion (15, 115, 215) in a laminar state when the magnetic bead extraction device operates.

8. The magnetic bead extraction device of one of the previous claims, wherein the width and height of the buffer solution chamber (20, 120, 220) are such that the buffer solution flows through the flow channel of the buffer solution chamber in a laminar state when the magnetic bead extraction device operates.

9. The magnetic bead extraction device of one of the previous claims, wherein the sample solution and the buffer solution flow in the same direction at the connecting portion.

10. A microfluidic system for separating target biomolecules from a sample solution using magnetic beads (51, 52), the microfluidic system comprising at least one magnetic bead extraction unit (100, 200) according to one of the previous claims.

11. The microfluidic system of claim 10, wherein the micro fluidic system comprises at least two magnetic bead extraction units (100, 200), the at least two magnetic bead extraction units being sequentially disposed such that a buffer solution chamber outlet of a first of the two magnetic bead extraction units is connected to a sample solution channel inlet (122) of a second of the two magnetic bead extraction units.

12. The microfluidic system of claim 10 or 11, wherein the magnetic beads have a diameter in the range of 0.001 µm to 200 µm.

13. The microfluidic system of one of claims 10 to 12, wherein the magnetic beads have a surface layer formed of a material selected from the group consisting of a metal oxide, styrene, agarose, and silica.

14. The microfluidic system of one of claims 10 to 13, wherein the magnetic beads have a probe thereon, the probe being capable of coupling with a particular target molecule.

15. The microfluidic system of claim 14, wherein the probe is one selected from the group consisting of an antibody, an antigen, a DNA, biotin, a protein, and streptavidin.

16. The microfluidic system of claim 14, wherein the probe comprises an amino radical (NH₂-) or a carboxyl radical (COOH-).
